# EUROPEAN PATENT SPECIFICATION

(11) **EP 2 397 465 B1**
(45) Date of publication and mention of the grant of the patent: **26.12.2012**
(21) Application number: 11168036.9
(22) Date of filing: 30.05.2011
(51) Int. Cl.: C07D 211/22

(54) **Fexofenadine polymorphs**
Polymorphen von Fexofenadin
Polymorphes de la fexofenadine

(30) Priority: 15.06.2010 IT MI20101078
(43) Date of publication of application: 21.12.2011
(73) Proprietor: Dipharma Francis S.r.l., 20021 Baranzate (MI) (IT)
(72) Inventor: Allegrini, Pietro, 20021 BARANZATE (MI) (IT); Razzetti, Gabriele, 20021 BARANZATE (MI) (IT)
(74) Representative: Minoja, Fabrizio

(56) References cited:
- EP-A1- 1 614 681
- WO-A1-95/31437
- US-A1- 2005 165 056

## Description

### FIELD OF THE INVENTION

The present invention relates to a process for the preparation of the polymorphic Form I of fexofenadine anhydrous hydrochloride known from WO 95/31437.

### TECHNOLOGICAL BACKGROUND

Fexofenadine hydrochloride (4-[4-[4-(hydroxydiphenylmethyl)-1-piperidinyl]-1-hydroxybutyl]-a,a-dimethylbenzeneacetic) acid hydrochloride of formula 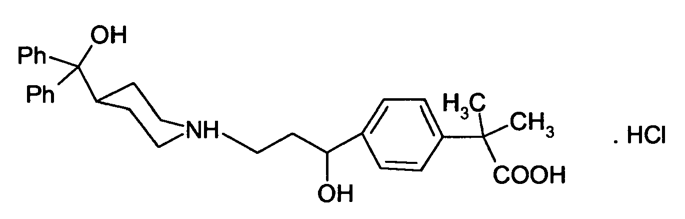 is an antihistamine and antiallergic drug, marketed in the U.S.A under the trade mark Allegra®.

WO 02/066429 discloses a non-hydrated polymorphic Form of fexofenadine hydrochloride (Form A).

EP 1614681 discloses the preparation of such Form A by a process comprising the formation of a crystalline Form of fexofenadine hydrochloride monosolvate with acetonitrile (Form C) and its conversion to the Form A. According to said method, fexofenadine hydrochloride Form C is obtained by a process comprising the preparation of a dispersion of fexofenadine hydrochloride hydrate (Form B) in acetonitrile, followed by heating at a temperature ranging from about 70°C to the reflux temperature for about an hour. The acetonitrile dispersion is then cooled to a temperature lower than -5°C.

The obtained precipitate of fexofenadine hydrochloride monosolvate Form C, is then isolated with known techniques. The removal of the acetonitrile solvent from said monosolvate Form C to obtain the anhydrous Form A can then be carried out with any physical means suitable to remove acetonitrile, preferably by heating under vacuum at about 80-110°C for a time ranging from about 18 to 30 hours.

WO 95/31437 describes hydrate and anhydrous forms of fexofenadine hydrochloride and it discloses, inter alia, the preparation of the anhydrous Form I from the hydrated Form II by azeotropical distillation or water-minimizing recrystallization. In particular, said recrystallization is carried out first forming a solution of the hydrated Form in an anhydrous solvent, and heating under reflux the solution, adding then either a further amount of the same solvent used to obtain the solution or a second suitable anhydrous anti-solvent to start precipitation of the anhydrous Form, then cooling the reaction mixture.

### SUMMARY OF THE INVENTION

It has now been found that fexofenadine hydrochloride anhydrous Form I can be prepared from fexofenadine hydrochloride monosolvate with acetonitrile in a simple, direct manner, with no need for such operations as azeotropical distillation, water-minimizing recrystallization, spray drying or freeze drying, thus avoiding the operations of filtration and washing of the filtrate, which would otherwise be required by the conversion process by recrystallization.

### BRIEF DISCLOSURE OF THE FIGURE

The crystalline anhydrous Form I was characterized using the known XRPD technique (X-ray powder diffraction). The X-ray diffraction spectrum (XRPD) was recorded using the APD 2000 θ/2θ automatic diffractometer for powders and liquids (Ital-Structures), under the following operative conditions: CuKα radiation (λ = 1.5418 Å), scanning with angular step of 0.03°C for a time 1 sec. The water content was determined by titration with the Karl Fischer technique.

Figure: XRPD spectrum (X-ray powder diffraction) of fexofenadine hydrochloride anhydrous Form I, wherein the most intense diffraction peaks fall at 5.9; 7.5; 12.2; 14.1; 15.0; 17.9; 18.3; 22.5; 23.5; and 26.7° in 2θ.

### DETAILED DISCLOSURE OF THE INVENTION

According to the present invention, "approximately monosolvate" means that the crystalline solid has a content in acetonitrile solvent of about 0.8-1.2 mols of solvent per mole of fexofenadine hydrochloride, preferably from about 0.9 to 1.1 mols.

According to the invention the expressions "precipitation" (or "precipitate") and crystallization (or "crystal") can be used indifferently and relate to the obtainment of a solid form.

According to a first aspect, the invention provides a process for the preparation of crystalline fexofenadine hydrochloride anhydrous Form I, as defined in WO 95/31437, comprising:
a) dispersing crystalline fexofenadine hydrochloride monosolvate with acetonitrile in an acetonitrile and water mixture;
b) seeding with crystalline fexofenadine hydrochloride anhydrous Form I;
c) refluxing the reaction mixture;
d) cooling the mixture;
e) separating and collecting the precipitate.

Preferably, the crystalline fexofenadine hydrochloride monosolvate with acetonitrile, used as starting material, is the Form C obtained according to EP 1614681.

The amount of water present in the acetonitrile mixture ranges from about 0.5 mole to 1.5 mole of fexofenadine hydrochloride monosolvate, in particular about 1 mole of water per mole of fexofenadine hydrochloride monosolvate.

The formation of a dispersion of crystalline fexofenadine hydrochloride monosolvate with acetonitrile in the acetonitrile and water mixture can be obtained by pouring said compound into said mixture or vice versa, at a temperature ranging from room temperature to about 50°C, in particular at about 25 - 40°C, in a weight/volume ratio ranging from about 1/8 to 1/15, preferably of about 1/10.

The seeding with crystalline fexofenadine hydrochloride anhydrous Form I is preferably carried out before starting refluxing of the reaction mixture. In any case, seeding can be carried out at any point after beginning heating.

Upon seeding the reaction mixture with crystalline fexofenadine hydrochloride anhydrous Form I, formation and precipitation of crystals of fexofenadine hydrochloride anhydrous Form I start.

The reaction mixture is kept under reflux temperature for a time ranging from about an hour and a half to five hours, preferably about three to four hours.

The subsequent cooling of the mixture is preferably carried out slowly, typically for a time ranging from about 15 minutes to 8 hours, preferably in about 4 - 5 hours, at a temperature ranging from about -20 to -5°C, preferably from about -15 to -10°C, and keeping the dispersion at this temperature for a time sufficient to obtain the complete precipitation of fexofenadine hydrochloride anhydrous Form I.

The precipitate is then separated and recovered with known techniques, for example by filtration or decantation of the solvent, preferably by filtration. The product is then dried at a temperature of about 60°C or below, preferably under vacuum at about 50°C for 10 hours.

According to a preferred aspect of the invention, crystalline fexofenadine hydrochloride monosolvate with acetonitrile (obtainable for example from crystalline fexofenadine hydrochloride hydrate according to EP 1614681) is not isolated and recovered as disclosed in EP 1614681, but its dispersion in acetonitrile is used as such at step b) of the process reported above.

The invention further comprises the formation of a dispersion of fexofenadine hydrochloride monosolvate with acetonitrile in acetonitrile, by a process comprising the formation of a dispersion of fexofenadine hydrochloride crystalline hydrate in acetonitrile and the conversion to crystalline fexofenadine hydrochloride monosolvate with acetonitrile.

The invention provides therefore a process for the preparation of crystalline fexofenadine hydrochloride anhydrous Form I, as defined in WO 95/31437, comprising:
a) dispersing fexofenadine hydrochloride crystalline hydrate in acetonitrile and converting it to fexofenadine hydrochloride monosolvate with acetonitrile;
b) seeding the dispersion with crystalline fexofenadine hydrochloride anhydrous Form I;
c) refluxing the reaction mixture;
d) cooling the mixture;
e) separating and collecting the precipitate.

The fexofenadine hydrochloride crystalline hydrate used as starting material, is preferably the crystalline Form monohydrate B, obtainable according to EP 1614681.

The formation of a dispersion of fexofenadine hydrochloride crystalline hydrate in acetonitrile can be obtained by pouring said product into warm acetonitrile, at about 35-60°C, preferably at about 40-55°C, in particular at about 45°C, in a weight/volume ratio ranging from about 1/8 to 1/15, preferably of about 1/10. Fexofenadine hydrochloride monosolvate with acetonitrile is obtained.

The subsequent steps of the process can be carried out as reported above for steps b) - e).

The preparation of crystalline fexofenadine hydrochloride anhydrous Form I, can be carried out already starting from fexofenadine hydrochloride hydrate, namely in the same reactor, with great operative advantages. Moreover, complex operations, such as completely dissolving of the starting fexofenadine, filtering and washing the filtrate, are no longer required, contrary to WO 95/31437.

The following examples illustrate the invention.

### Example 1: Preparation of fexofenadine hydrochloride Form B

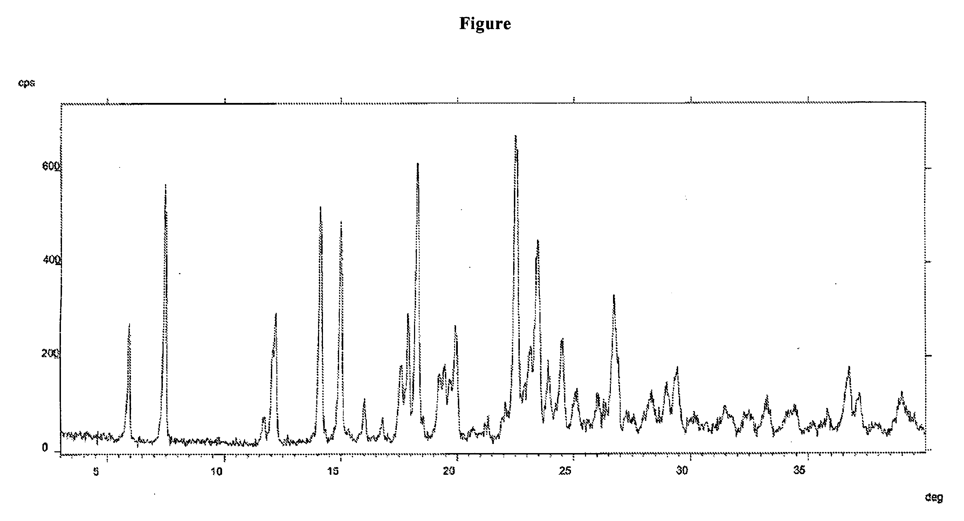

278 g of 4-[4-[4-(hydroxydiphenylmethyl)-1-piperidinyl]-1-oxobutyl]-α,α-dimethylbenzeneacetic acid, 355 ml of water, 765 ml of methanol and 22.6 g of sodium hydroxide scales are loaded into a 3 liter four-necked flask. The resulting mixture is heated at 40°C, then a solution of sodium borohydride (9.8 g) in 47 ml of water is added. After completion of the reduction reaction, 40 ml of acetone are added and pH is adjusted to a value ranging from 2.5 to 3 with 36% w/w hydrochloric acid. 140 ml of methanol are added, then 450 ml of water, keeping the temperature at 40°C. The resulting mixture is slowly cooled to -15°C and the formed precipitate is filtered and washed with a water and methanol mixture in 1/1 ratio. The resulting product is dried under vacuum at 40°C to constant weight. 265 g of fexofenadine hydrochloride are obtained with 4.1% water content according to Karl-Fischer, so that it can be defined monohydrate, having an XRPD spectrum as illustrated in Figure 1 of EP 1614681, wherein the most intense diffraction peaks fall at 23.6; 10.1; 7.9; 5.2; 4.7 and 4.4 in 2θ.

### Example 2: Preparation of fexofenadine hydrochloride Form C

2650 ml of acetonitrile are loaded into a 3 liter four-necked flask and heated at 45°C. Then 265 g of fexofenadine hydrochloride hydrate, as obtainable from Example 1, are loaded under stirring and the suspension is refluxed (80-82°C) keeping said conditions for about an hour. The suspension is cooled to about -15, -10°C in about 4 hours. The solid is filtered and washed with acetonitrile (2 x 80 ml). 285 g of wet product are obtained which, after drying under vacuum at 50°C for 10 hours, yields 262 g of fexofenadine hydrochloride monosolvate with acetonitrile, having an XRPD spectrum as illustrated in Figure 3 of EP 1614681, wherein the most intense diffraction peaks fall at 7.0; 11.6; 15.4; 17.3; 18.0 and 20.5 in 2θ; and 0.25% water content according to Karl-Fischer.

### Example 3: Preparation of fexofenadine hydrochloride Form I (from crystalline fexofenadine hydrochloride monosolvate with acetonitrile)

2650 ml of acetonitrile, 13 g of water and 262 g of fexofenadine hydrochloride monosolvate with acetonitrile, as obtainable from Example 2, are loaded into a 3 liter four-necked flask. 100 mg of fexofenadine hydrochloride Form I are added and the suspension is refluxed (80-82°C), keeping said conditions for about 3 hours. The suspension is cooled to about -15, -10°C in about 4 hours. The solid is filtered and washed with acetonitrile (2 x 80 ml). 267 g of wet product are obtained which, after drying under vacuum at 50°C for 10 hours, yields 236 g of fexofenadine hydrochloride Form I, having an XRPD spectrum as illustrated in the Figure, wherein the most intense diffraction peaks fall at 5.9; 7.5; 12.2; 14.1, 15.0; 17.9; 18.3; 22.5; 23.5; and 26.7° in 2θ; and 0.12% water content according to Karl-Fischer.

### Example 4: Preparation of fexofenadine hydrochloride Form I (from fexofenadine hydrated Form)

1325 ml of acetonitrile are loaded into a 3 liter four-necked flask and heated at 45°C. Then 133 g of fexofenadine hydrochloride hydrate, as obtainable from Example 1, are loaded under stirring, 100 mg of fexofenadine hydrochloride Form I are added and the suspension is refluxed (80-82°C) keeping said conditions for about 3 hours. The suspension is cooled to about -15, -10°C in about 4 hours. The solid is filtered and washed with acetonitrile (2 x 40 ml). 140 g of wet product are obtained which, after drying under vacuum at 50°C for 10 hours, yields 120 g of fexofenadine hydrochloride Form I, having an XRPD spectrum as illustrated in the Figure, wherein the most intense diffraction peaks fall at 5.9; 7.5; 12.2; 14.1, 15.0; 17.9; 18.3; 22.5; 23.5; and 26.7° in 2θ; and 0.11% water content according to Karl-Fischer.

## Claims

1. A process for preparing crystalline fexofenadine hydrochloride anhydrous Form I wherein the most intense diffraction peaks fall at 5.9; 7.5; 12.2; 14.1, 15.0; 17.9; 18.3; 22.5; 23.5; and 26.7° in 2θ, comprising:
a) dispersing crystalline fexofenadine hydrochloride monosolvate with acetonitrile in a mixture of acetonitrile and water;
b) seeding with crystalline fexofenadine hydrochloride anhydrous Form I;
c) refluxing the reaction mixture;
d) cooling the mixture;
e) separating and collecting the precipitate.

2. Process according to claim 1, wherein the crystalline fexofenadine hydrochloride monosolvate with acetonitrile, is Form C wherein the most intense diffraction peaks fall at 7.0; 11.6; 15.4; 17.3; 18.0 and 20.5 in 2θ.

3. Process according to claim 1, wherein the amount of water in the mixture with acetonitrile ranges from about 0.5 mole to 1.5 mole per mole of fexofenadine hydrochloride, preferably about one mole of water per mole of fexofenadine hydrochloride monosolvate with acetonitrile.

4. Process according to claim 1, wherein the weight/volume ratio between crystalline fexofenadine hydrochloride monosolvate with acetonitrile and the mixture of acetonitrile and water is about from 1/8 and 1/15, preferably about 1/10.

5. Process according to claim 1, wherein seeding with crystalline fexofenadine hydrochloride anhydrous Form I is carried out before heating the reaction mixture to reflux temperature.

6. Process according to claim 1, wherein the reaction mixture is refluxed for a time ranging from about 1.5 to 5 hours, preferably from about 3 to 4 hours.

7. Process according to claim 1, wherein the mixture is slowly cooled, preferably in a time ranging from about 15 minutes to 8 hours, preferably in about 4 - 5 hours.

8. Process according to claim 1, wherein the mixture is cooled at a temperature between about -20 and -5°C, preferably between about -15 and -10°C.

9. Process according to claim 1, further comprising dispersing crystalline fexofenadine hydrochloride monosolvate with acetonitrile in a mixture of acetonitrile and water, by a process comprising dispersing hydrate crystalline fexofenadine hydrochloride in acetonitrile and converting it into crystalline fexofenadine hydrochloride monosolvate with acetonitrile.

10. Process according to claim 9 wherein fexofenadine hydrochloride monosolvate with acetonitrile is Form C having the most intense diffraction peaks falling at 7.0; 11.6; 15.4; 17.3; 18.0 and 20.5 in 2θ and hydrate crystalline fexofenadine hydrochloride is Form B having the most intense diffraction peaks falling at 23.6; 10.1; 7.9; 5.2; 4.7 and 4.4 in 2θ.

11. A process for preparing anhydrous crystalline fexofenadine hydrochloride Form I wherein the most intense diffraction peaks fall at 5.9; 7.5; 12.2; 14.1, 15.0; 17.9; 18.3; 22.5; 23.5; and 26.7° in 2θ, comprising:
a) providing a dispersion of hydrate crystalline fexofenadine hydrochloride in acetonitrile and its conversion into crystalline fexofenadine hydrochloride monosolvate with acetonitrile;
b) seeding with crystalline fexofenadine hydrochloride anhydrous Form I;
c) refluxing the reaction mixture;
d) cooling the mixture;
e) separating and collecting the precipitate.

12. Process according to claim 11 wherein fexofenadine hydrochloride monosolvate with acetonitrile is Form C having the most intense diffraction peaks falling at 7.0; 11.6; 15.4; 17.3; 18.0 and 20.5 in 2θ and hydrate crystalline fexofenadine hydrochloride is Form B having the most intense diffraction peaks falling at 23.6; 10.1; 7.9; 5.2; 4.7 and 4.4 in 2θ.

## Patentansprüche

1. Verfahren zur Herstellung einer wasserfreien kristallinen Fexofenadinhydrochlorid Form 1, worin die stärksten Beugungspeaks bei 5,9; 7,5; 12,2; 14,1; 15,0; 17,9; 18,3; 22,5; 23,5 und 26,7° bei 2θ auftreten, umfassend:
a) Dispergleren von kristallinem Fexofenadinhydrochloridmonosolvat mit Acetonitril in einer Mischung von Acetonitril und Wasser;
b) Impfen mit wasserfreier kristalliner Fexofenadlnhydrochlorid Form I;
c) Refluxieren der Reaktionsmischung;
d) Abkühlen der Mischung;
e) Abtrennen und Sammeln des Niederschlags.

2. Verfahren gemäß Anspruch 1, worin das kristalline Fexofenadinhydrochloridmonosolvat mit Acetonitril die Form C ist, worin die stärksten Beugungspeaks bei 7,0; 11,6; 15,4; 17,3; 18,0 und 20,5 bei 2θ auftreten.

3. Verfahren gemäß Anspruch 1, worin der Gehalt von Wasser in der Mischung mit Acetonitril von etwa 0,5 Mol bis 1,5 Mol pro Mol Fexofenadinhydrochlorid beträgt, bevorzugt etwa ein Mol Wasser pro Mol Fexofenadinhydrochloridmonosolvat mit Acetonitril.

4. Verfahren gemäß Anspruch 1, worin das Gewicht/VolumenVerhältnis zwischen kristallinem Fexofenadinhydrochloridmonosolvat mit Acetonitril und der Mischung von Acetonitril und Wasser etwa zwischen 1/8 und 1/15, bevorzugt etwa 1/10 beträgt.

5. Verfahren gemäß Anspruch 1, worin das Impfen mit wasserfreier kristalliner Fexofenadinhydrochlorid Form I vor dem Erhitzen der Reaktionsmischung auf die Rückflusstemperatur durchgeführt wird.

6. Verfahren gemäß Anspruch 1, worin die Reaktionsmischung für eine Zeit von etwa 1,5 bis 5 Stunden, bevorzugt von etwa 3 bis 4 Stunden refluxiert wird.

7. Verfahren gemäß Anspruch 1, worin die Mischung langsam abgekühlt wird, bevorzugt in einer Zeit von etwa 15 Minuten bis 8 Stunden, bevorzugt in etwa 4 bis 5 Stunden.

8. Verfahren gemäß Anspruch 1, worin die Mischung bei einer Temperatur zwischen etwa -20 und -5 °C, bevorzugt zwischen etwa -15 und -10 °C abgekühlt wird.

9. Verfahren gemäß Anspruch 1, welches außerdem das Dispergieren von kristallinem Fexofenadinhydrochlorldmonosolvat mit Acetonitril in einer Mischung von Acetonitril und Wasser durch ein Verfahren umfassend das Dispergieren von kristallinem Fexofenadinhydrochlorldhydrat in Acetonitril und dessen Umwandlung in kristallines Fexofenadinhydrochloridmonosolvat mit Acetonitril umfasst.

10. Verfahren gemäß Anspruch 9, worin das Fexofenadinhydrochloridmonosolvat mit Acetonitril Form C ist, worin die stärksten Beugungspeaks bei 7,0; 11,6; 15,4; 17,3; 18,0 und 20,5 bei 2θ auftreten und das kristalline Fexofienadinhydrochloridhydrat Form B ist, worin die stärksten Beugungspeaks bei 23,6; 10,1; 7,9; 5,2; 4,7 und 4,4 bei 2θ auftreten.

11. Verfahren zur Herstellung von wasserfreier kristalliner Fexofienadinhydrochlorid Form 1, worin die stärksten Beugungspeaks bei 5,9; 7,5; 12,2; 14,1; 15,0; 17,9; 18,3; 22,5; 23,5 und 26,7° bei 20 auftreten, umfassend:
a) Bereitstellen einer Dispersion von kristallinem Fexofenadinhydrochloridhydrat in Acetonitril und Umwandlung desselben in kristallines Fexofenadinhydrochloridmonosolvat mit Acetonitril,
b) Impfen mit wasserfreler kristalliner Fexofenadinhydrochlorid Form I;
c) Refluxieren der Reaktionsmischung;
d) Abkühlen der Mischung;
e) Abtrennen und Sammeln des Niederschlags,

12. Verfahren gemäß Anspruch 11, worin das Fexofenadinhydrochlorldmonosolvat mit Acetonitril Form C ist, worin die stärksten Beugungspeaks bei 7,0; 11,6; 15,4; 17,3; 18,0 und 20,5 bei 2θ auftreten und das kristalline Fexofenadinhydrochlorldhydrat Form B ist, worin die stärksten Beugungspeaks bei 23,6; 10,1; 7,9; 5,2; 4,7 und 4,4 bei 2θ auftreten.

## Revendications

1. Procédé pour préparer la forme I anhydre du chlorhydrate cristallin de fexofénadine dans laquelle les pics de diffraction les plus intenses tombent à 5,9 ; 7,5 ; 12,2 ; 14,1 ; 15,0 ; 17,9 ; 18,3 ; 22,5 ; 23,5 et 26,7° dans 2θ, comprenant :
a) la mise en dispersion du monosolvate du chlorhydrate cristallin de fexofénadine avec de l'acétonitrile dans un mélange d'acétonitrile et d'eau ;
b) l'ensemencement avec la forme I anhydre du chlorhydrate cristallin de fexofénadine ;
c) le chauffage à reflux du mélange réactionnel ;
d) le refroidissement du mélange ;
e) la séparation et la récolte du précipité.

2. Procédé selon la revendication 1, dans lequel le monosolvate du chlorhydrate cristallin de fexofénadine est la forme C dans laquelle les pics de diffraction les plus intenses tombent à 7,0 ; 11,6 ; 15,4 ; 17,3 ; 18,0 et 20,5 dans 2θ.

3. Procédé selon la revendication 1, dans lequel la quantité d'eau dans le mélange avec l'acétonitrile se situe dans la plage d'environ 0,5 mole à 1,5 mole par mole de chlorhydrate de fexofénadine, de préférence d'environ 1 mole d'eau par mole de monosolvate du chlorhydrate de fexofénadine avec l'acétonitrile.

4. Procédé selon la revendication 1, dans lequel le rapport poids/volume entre le monosolvate du chlorhydrate cristallin de fexofénadine avec l'acétonitrile et le mélange d'acétonitrile et d'eau se situe entre environ 1/8 et 1/15, de préférence s'élève à environ 1/10.

5. Procédé selon la revendication 1, dans lequel l'ensemencement avec la forme I anhydre du chlorhydrate cristallin de fexofénadine est mis en oeuvre avant de chauffer le mélange réactionnel jusqu'à la température de reflux.

6. Procédé selon la revendication 1, dans lequel le mélange réactionnel est chauffé à reflux pendant un laps de temps se situant dans la plage d'environ 1,5 à 5 heures, de préférence d'environ 3 à 4 heures.

7. Procédé selon la revendication 1, dans lequel on refroidit le mélange lentement, de préférence dans un laps de temps se situant dans la plage d'environ 15 minutes à 8 heures, de préférence dans un laps de temps d'environ 4 à 5 heures.

8. Procédé selon la revendication 1, dans lequel on refroidit le mélange à une température entre -20 et - 5 °C, de préférence entre environ -15 et -10 °C.

9. Procédé selon la revendication 1, comprenant en outre la mise en dispersion du monosolvate du chlorhydrate cristallin de fexofénadine avec de l'acétonitrile dans un mélange d'acétonitrile et d'eau, via un procédé comprenant la mise en dispersion du chlorhydrate cristallin hydraté de fexofénadine dans de l'acétonitrile et sa transformation en monosolvate du chlorhydrate cristallin de fexofénadine avec de l'acétonitrile.

10. Procédé selon la revendication 9, dans lequel le monosolvate du chlorhydrate de fexofénadine avec de l'acétonitrile est la forme C dont les pics de diffraction les plus intenses tombent à 7,0 ; 11,6 ; 15,4 ; 17,3 ; 18,0 et 20,5 dans 2θ et le chlorhydrate cristallin hydraté de fexofénadine est la forme B dont les pics de diffraction les plus intenses tombent à 23,6 ; 10,1 ; 7,9 ; 5,2 ; 4,7 et 4,4 dans 2θ.

11. Procédé pour préparer la forme I anhydre du chlorhydrate cristallin de fexofénadine dans laquelle les pics de diffraction les plus intenses tombent à 5,9 ; 7,5 ; 12,2 ; 14,1 ; 15,0 ; 17,9 ; 18,3 ; 22,5 ; 23,5 et 26,7° dans 2θ, comprenant :
a) la mise à disposition d'une dispersion du chlorhydrate cristallin hydraté de fexofénadine et sa conversion en monosolvate du chlorhydrate cristallin de fexofénadine avec de l'acétonitrile ;
b) l'ensemencement avec la forme I anhydre du chlorhydrate cristallin de fexofénadine ;
c) le chauffage à reflux du mélange réactionnel ;
d) le refroidissement du mélange ;
e) la séparation et la récolte du précipité.

12. Procédé selon la revendication 11, dans lequel le monosolvate du chlorhydrate de fexofénadine avec de l'acétonitrile est la forme C dont les pics de diffraction les plus intenses tombent à 7,0 ; 11,6 ; 15,4 ; 17,3 ; 18,0 et 20,5 dans 2θ et le chlorhydrate cristallin hydraté de fexofénadine est la forme B dont les pics de diffraction les plus intenses tombent à 23,6 ; 10,1 ; 7,9 ; 5,2 ; 4,7 et 4,4 dans 2θ.
